# EUROPÄISCHE PATENTSCHRIFT

(11) **EP 0 573 763 B1**
(45) Veröffentlichungstag und Bekanntmachung des Hinweises auf die Patenterteilung: **03.07.1996**
(21) Anmeldenummer: 93106680.7
(22) Anmeldetag: 24.04.1993
(51) Int. Cl.: C07C 67/08, C07C 69/675

(54) **Verfahren zur Herstellung von Hydroxypivalinsäureestern**
Process for the preparation of hydroxy pivalic acid esters
Procédé de préparation d'esters de l'acide hydroxypilaloique

(30) Priorität: 12.05.1992 DE 4215494
(43) Veröffentlichungstag der Anmeldung: 15.12.1993
(73) Patentinhaber: BASF Aktiengesellschaft, D-67063 Ludwigshafen (DE)
(72) Erfinder: Merger, Franz, Dr., W-6710 Frankenthal (DE); Schmidt-Radde, Martin, Dr., W-6711 Beindersheim (DE)

(56) Entgegenhaltungen:
- EP-A- 0 360 087
- EP-A- 0 410 167
- DE-A- 3 045 373
- DE-A- 3 638 009

## Beschreibung

Die vorliegende Erfindung betrifft ein neues Verfahren zur Herstellung von Hydroxypivalinsäureestern der allgemeinen Formel I 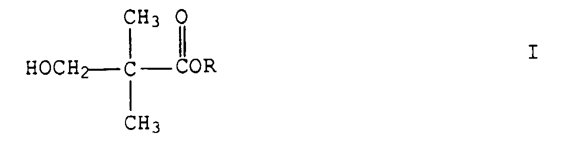 in der R für eine C₁-C₄-Alkyl- oder Alkenylgruppe steht.

Bei der Herstellung von Neopentylglycol (III) aus Isobutyraldehyd und Formaldehyd und der anschließenden Hydrierung sowie der destillativen Abtrennung vom III fällt ein Destillationsrückstand an, der je nach den Synthesebedingungen verschiedene Mengen an Hydroxypivalinsäure (II), Neopentylglycol 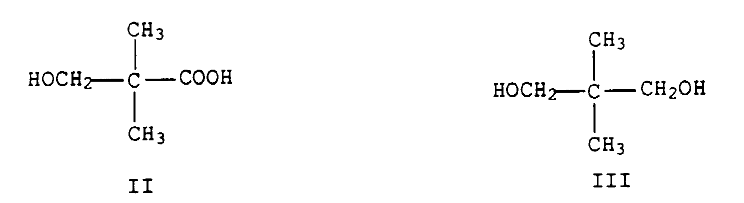 und Estern (IV) aus II und III enthält.

In der EP-A 410 167 wird ein Verfahren zur Aufarbeitung solcher Gemische vorgeschlagen, indem man II und III zum Hydroxypivalinsäureneopentylglycolester IVa verestert. Liegen im Ausgangsmaterial II und III jedoch nicht in etwa äquimolaren Mengen vor, erhält man ein Gemisch aus IV und II bzw. III, von welchen sich IVa nur schwierig abtrennen läßt 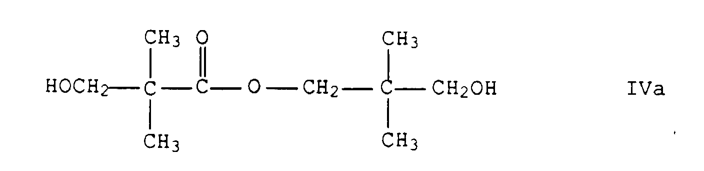 Aus der DE-A 36 38 009 ist die Umesterung von IVa in Gegenwart eines Katalysators mit einem Alkohol zu den entsprechenden Hydroxypivalinsäureestern bekannt.

Der Erfindung lag die Aufgabe zugrunde, aus den genannten Destillationsrückständen unabhängig von deren mengenmäßiger Zusammensetzung auf einfache und wirtschaftliche Weise Wertprodukte zu gewinnen.

Demgemäß wurde ein neues Verfahren zur Herstellung von Hydroxypivalinsäureestern I gefunden, das dadurch gekennzeichnet ist, daß man ein Gemisch, welches Hydroxypivalinsäure (II), Neopentylglycol (III) sowie verschiedene Ester (IV) aus II und III enthält, zur Veresterung von II und III mit katalytischen Mengen einer Säure behandelt, die stärker ist als II, das hierbei entstehende Gemisch in Gegenwart einer starken Base der Umesterung mit einem Alkohol R-OH (V) unterwirft und I danach destillativ vom Reaktionsgemisch abtrennt.

Im folgenden sollen die besonders bevorzugten Ausführungsformen der Erfindung erläutert werden.

Die erfindungsgemäß aufzuarbeitenden Gemische können II, III sowie Ester IV in beliebigen Mengenverhältnissen enthalten. Typische Gemische dieser Art enthalten
10 bis 20 Gew.-% II,
5 bis 10 Gew.-% III,
40 bis 50 Gew.-% IVa und
20 bis 45 Gew.-% höhermolekulare Ester IV.

Als saure Katalysatoren für die Veresterung kommen organische Säuren, die stärker sauer sind als II, wie para-Toluolsulfonsäure, und Mineralsäuren wie Phosphorsäure oder bevorzugt Schwefelsäure in Betracht. Die Mineralsäuren können dem Reaktionsgemisch in freier Form oder an Trägermaterialien adsobiert zugesetzt werden. Bevorzugtes Trägermaterial ist SiO₂. In einer besonders bevorzugten Ausführungsform besteht der Katalysator aus einem SiO₂-Träger, der mit 20 Gew.-% Schwefelsäure imprägniert ist. Dieser Katalysator kann in Form von Strängen oder Pulvern eingesetzt werden.

Weiterhin sind als Katalysatoren Ionentauscher zu nennen, die Sulfonsäuregruppen tragen.

Die sauren Verbindungen werden in katalytisch wirksamen Mengen eingesetzt. Bevorzugt werden 0,2 bis 5 Gew.-%, besonders bevorzugt werden 0,5 bis 4 Gew.-% Säure, bezogen auf die Menge des umzusetzenden Gemisches aus II, III und IV verwendet.

Der Katalysator und das umzusetzende Gemisch werden vorzugsweise bei etwa 120 bis 200°C, besonders bevorzugt bei 130 bis 160°C umgesetzt. Man kann die Reaktion bei Normaldruck ausführen. Vorzugsweie arbeitet man aber bei einem verminderten Druck von etwa 50 bis 200 mbar. Während der Reaktion wird in einer bevorzugten Ausführungsform das bei der Veresterung gebildete Wasser abdestilliert. Die Reaktionsdauer liegt je nach Art und Menge des Katalysators üblicherweise bei 0,5 bis 12 Stunden. Die Reaktion kann als beendet gelten, wenn der Gehalt an II im Gemisch auf unter 2 Gew.-% gefallen ist.

Das so erhaltene Reaktionsgemisch wird einer Umesterung unterworfen.

Dazu werden starke Basen als Umesterungskatalysatoren verwendet. Es kommen vorzugsweise Alkalimetall- und Erdalkalimetallalkoholate wie Calcium- oder Magnesiumalkoholate in Betracht, besonders bevorzugt werden aber Kalium- und Natriumalkoholate.

Vorzugsweise setzt man in der Reaktion Alkoholate von Alkoholen mit 1 bis 6 Kohlenstoffatomen ein, besonders bevorzugt sind aber die Alkoholate der bei der Umesterung verwendeten Alkohole R-OH V.

Die Katalysatormenge beträgt in der Regel 0,5 bis 5 Gew.-%, vorzugsweise 1,5 bis 3 Gew.-%, bezogen auf die Menge des umzuesternden Reaktionsgemisches.

Die Alkohole R-OH V, die für die Umesterung verwendet werden, enthalten bis zu 4 Kohlenstoffatome. Sie umfassen u.a. Methanol, Ethanol, iso-Propanol, n-Propanol, n-Butanol sowie ungesättigte Alkohole wie Allylalkohol. Methanol ist besonders bevorzugt.

Die Alkoholmenge beträgt üblicherweise 50 bis 500, vorzugsweise 100 bis 300 Gew.-% des umzuesternden Reaktionsgemisches. Größere Alkoholmengen sind möglich, bringen aber keine Vorteile.

Man kann das umzuesternde Reaktionsgemisch, den Alkohol und den Umesterungskatalysator mischen und die Reaktion dann vorzugsweise bei 20 bis 100°C bei Normaldruck über 0,5 bis 10 h ausführen. Unter diesen Bedingungen stellt sich üblicherweise das Umesterungsgleichgewicht ein. Höhere Temperaturen und Drücke sind möglich, bieten aber keine Vorteile.

Nach der Umesterung wird das Reaktionsgemisch mit einer organischen Säure wie Ameisensäure oder einer anorganischen Säure wie Schwefelsäure neutralisiert. Die Reaktionsprodukte werden dann in an sich bekannter Weise destillativ isoliert und gereinigt.

Das Verfahren ermöglicht es, Wertprodukte aus Gemischen von II, III und Estern aus II und III in hoher Ausbeute zu gewinnen, wobei die molare Zusammensetzung der Gemische eine untergeordnete Rollte spielt. Neopentylglycol III wird in hoher Ausbeute zurückgewonnen.

Erfindungsgemäß werden Hydroxypivalinsäureester I gewonnen, die als Ausgangsstoffe für Pflanzenschutzmittel (DE-A 36 38 009) dienen oder direkt als Bausteine in Alkydharzen Verwendung finden.

### Beispiel

### Herstellung von Hydroxypivalinsäuremethylester (Ia)

Als Ausgangsgemisch wurden 600 g der Sumpffraktion einer Neopentylglykoldestillation folgender Zusammensetzung
16 Gew.-% Hydroxypivalinsäure (II)
7 Gew.-% Neopentylglycol (III)
43 Gew.-% des Monoesters aus II und III (IVa) und
34 Gew.-% höherer Ester IV
eingesetzt.

Das Gemisch wurde mit 54 g eines sauren Katalysators aus 80 Gew.-% SiO₂ und 20 Gew.-% Schwefelsäure unter einem Druck von 100 mbar 3 Stunden lang auf 140°C erhitzt, wobei das Reaktionswasser abdestilliert wurde. Dabei destillierten 16 g Wasser ab.

Das so erhaltene Reaktionsgemisch wurde mit 1440 g Methanol und 45 g 30 gew.-%iger methanolischer Natriummethylatlösung 3 h auf 50°C erhitzt. Nach Neutralisation und Destillation erhielt man Ia in 81 % Ausbeute und III in 78 % Ausbeute, jeweils bezogen auf die Anteile und Strukturanteile dieser Verbindungen im ursprünglichen Gemisch.

## Patentansprüche

1. Verfahren zur Herstellung von Hydroxypivalinsäureestern der allgemeinen Formel I 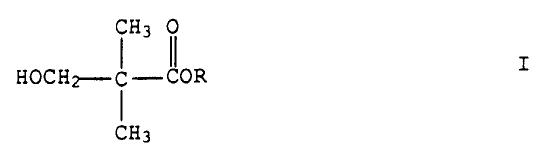 in der R für eine C₁-C₄-Alkyl- oder Alkenylgruppe steht, dadurch gekennzeichnet, daß man ein Gemisch, welches Hydroxypivalinsäure (II), Neopentylglycol (III) sowie verschiedene Ester (IV) aus II und III enthält, zur Veresterung von II und III mit katalytischen Mengen einer Säure behandelt, die stärker ist als II, das hierbei entstehende Gemisch in Gegenwart einer starken Base der Umesterung mit einem Alkohol R-OH (V) unterwirft und I danach destillativ vom Reaktionsgemisch abtrennt.

2. Verfahren nach Anspruch 1, dadurch gekennzeichnet, daß man Hydroxypivalinsäuremethylester herstellt.

## Claims

1. A process for preparing hydroxypivalic esters of the general formula I 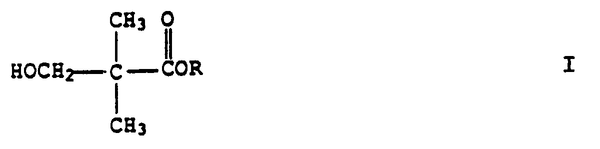 where R is C₁-C₄-alkyl or alkenyl, which comprises treating a mixture which contains hydroxypivalic acid (II), neopentyl glycol (III) and various esters (IV) of II and III, for the esterification of II and III, with catalytic amounts of an acid which is stronger than II, subjecting the resulting mixture to transesterification with an alcohol R-OH (V) in the presence of a strong base, and then removing I from the reaction mixture by distillation.

2. A process as claimed in claim 1, wherein methyl hydroxypivalate is prepared.

## Revendications

1. Procédé de préparation d'esters de l'acide hydroxypivalique de la formule générale I 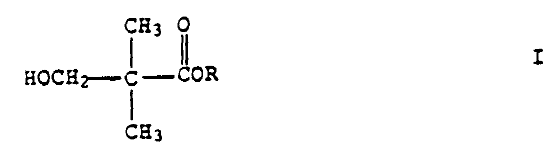 dans laquelle R représente un radical alcényle ou alkyle en C₁ à C₄ ce que l'on traite un mélange, qui contient de l'acide hydroxypivalique (II), du néopentylglycol (III), ainsi que divers esters (IV) de (II) et de (III), en vue de l'estérification de (II) et de (III), par des proportions catalytiques d'un acide, qui est plus fort que (II), on soumet le mélange ainsi obtenu, en présence d'une base forte, à la transestérification avec un alcool R-OH (V) et on sépare ensuite (I) du mélange de réaction par distillation.

2. Procédé suivant la revendication 1, caractérisé en ce que l'on prépare l'hydroxypivalate de méthyle.
